# EUROPÄISCHE PATENTSCHRIFT

(11) **EP 3 669 857 B1**
(45) Veröffentlichungstag und Bekanntmachung des Hinweises auf die Patenterteilung: **30.10.2024**
(21) Anmeldenummer: 19211837.0
(22) Anmeldetag: 27.11.2019
(51) Int. Cl.: A61K 8/02, A61K 8/365, A61K 8/44, A61K 8/46, A61K 8/49, A61Q 5/12, A61K 8/19

(54) **PULVERFÖRMIGER HAARPFLEGEBOOSTER MIT ORGANISCHER SÄURE**
POWDERED HAIR CARE BOOSTER WITH ORGANIC ACID
BOOSTER DE SOIN DES CHEVEUX EN POUDRE COMPRENANT UN ACIDE ORGANIQUE

(30) Priorität: 18.12.2018 DE 102018222060
(43) Veröffentlichungstag der Anmeldung: 24.06.2020
(73) Patentinhaber: Henkel AG & Co. KGaA, 40589 Düsseldorf (DE)
(72) Erfinder: Ahnfeldt, Tina, 22547 Hamburg (DE); Kerl, Sylvia, 25474 Bönningstedt (DE); von Aspern, Edith, 21271 Hanstedt (DE)

(56) Entgegenhaltungen:
- CN-A- 108 714 124
- DE-A1- 102012 220 807
- KR-A- 20130 032 927
- IRIT BAR-YA'AKOV ET AL: "Primary Metabolites, Anthocyanins, and Hydrolyzable Tannins in the Pomegranate Fruit", FRONTIERS IN PLANT SCIENCE, vol. 10, 17 May 2019 (2019-05-17), XP055668556, DOI: 10.3389/fpls.2019.00620
- ZHU SHAOQUING ET AL: "UHPLC-TQ-MS Coupled with Multivariate Statistical Analysis to Characterize Nucleosides, Nucleobases and Amino Acids in Angelicae Sinensis Radix Obtained by Different Drying Methods", MOLECULES ONLINE, vol. 22, no. 6, 1 June 2017 (2017-06-01), DE, pages 918, XP055669356, ISSN: 1433-1373, DOI: 10.3390/molecules22060918
- DATABASE WPI Week 201351, Derwent World Patents Index; AN 2013-F47629
- DATABASE GNPD [online] MINTEL; 26 April 2018 (2018-04-26), ANONYMOUS: "Bamboom Volumizing & Cleansing Powder", XP055668548, retrieved from www.gnpd.com Database accession no. 5621527
- DATABASE GNPD [online] MINTEL; 12 April 2016 (2016-04-12), ANONYMOUS: "BB Mineral Lucent Powder", XP055668547, retrieved from www.gnpd.com Database accession no. 4235357
- DATABASE GNPD [online] MINTEL; 4 January 2016 (2016-01-04), ANONYMOUS: "Facial Washing Powder", XP055668521, retrieved from www.gnpd.com Database accession no. 3704149

## Beschreibung

Ein heller, insbesondere blonder Haarfarbton wird von vielen Menschen als schön und attraktiv wahrgenommen. Jedoch kann die helle und strahlende Erscheinung eines solchen Farbton durch verschiedene Faktoren beeinträchtigt werden, unter anderem durch An- und Ab- und/oder Einlagerunen von Fremdstoffen, beispielsweise aus Haarpflegeprodukten und oder Haarreinigungsprodukten. Andere Quellen von Ablagerungen and den Haaren können ebenfalls eine Rolle spielen, beispielsweise Hautpflegeprodukte, in Wasser, wie Leitungs- oder Schwimmbadwasser, gelöste, dispergierte oder in anderer Form vorliegende Stoffe, sowie in der Luft beispielsweise als Aerosole vorliegende Stoffe. Je nach individueller Haarausprägung und lokalen Umgebungsbedingungen entsteht insgesamt in viele Fällen eine etwas unattraktive Ablagerung an Haaren, die auf hellem Haar, insbesondere auf hellblondem Haar einen negativen optischen Eindruck hervorrufen kann.

Insgesamt wird blonden Haaren ein hohes Pflegebedürfnis attestiert. Trockene Spitzen, Gelbstich oder Farbverlust sind einige verbreitete Probleme, die nach Lehrmeinung bei blondem Haar möglicherweise verstärkt auftreten und denen sich blondhaarige Personen mithin immer wieder stellen müssen. Der Wunsch vieler Menschen nach einem gepflegten Blond als "Eye Catcher" ist andererseits weit verbreitet. Dies führt zu einer verbreiteten Verwendung von Aufhellungs- und Blondierungsprodukten, insbesondere bei Frauen denn selbst in vielen Westlichen Kulturkreisen sind nur zwei Prozent aller Frauen von Natur aus wirklich hellblond, die meisten Blonden Haare sind asch- oder dunkelblond. Um die wahrgenommen optimale Haarfarbe zu kreieren, wird also blondiert. Dabei wird die Schuppenschicht der Haare chemisch geöffnet, damit die natürlichen Farbpigmente austreten können. Das Ergebnis sind jedoch meist spröde und trockene Haare.

Auch mit zunehmender Haarlänge werden diese Probleme bedeutender, da die Haare über einen längeren Zeitraum verschiedenen Belastungen, wie zuvor beschrieben, ausgesetzt sind. Besonderer Bedarf an speziellen und besonders guten Pflege und Reinigungsprodukten besteht also bei Anwenderinnen oder Anwendern mit langen blondierten Haaren.

Die hier beschriebene Erfindung wurde im Rahmen von Forschungsarbeiten zu Adressieren der zuvor geschilderten Problems gemacht. Sie betrifft einen Haarpflegebooster, also ein Produkt, das einem herkömmlichen Haarpflegeprodukt zugesetzt werden kann, um bestimmte dessen Pflege- und oder - Reinigungsleistung im Hinblick auf bestimmte Parameter, hier vor allem Beseitigen von Ablagerungen von blondiertem Haar zu verbessern.

Herkömmlicher Weise werden für den gleichen Zweck spezielle Reinigungs- und Pflegeprodukte empfohlen, wie sie beispielsweise auch für grau oder weiß gewordenes Haar entwickelt wurden, insbesondere sogenannte Silbershampoos oder spezielle Produkte mit bestimmten pflanzlichen, denen in dieser Hinsicht vorteilhafte Wirkungen zugeschrieben werden. CN108714124A offenbart ein Haarpflegeprodukt in Pulverform enthaltend pflanzliche Bestandteile wie z.B. Extrakt von Angelica sinensis. Das Produkt wird mit Wasser vermischt und an den Haaren angewendet.

Die Wirkung der Verfügbaren Produkte wird jedoch verbreitet als nicht zufriedenstellen angesehen und es gibt regen Austausch unter Anwendern mit "do-it-yourself"-Verbesserungsvorschlägen. Es wird also ein Bedarf für bessere Produkte für die zuvor beschriebene Anmeldung wahrgenommen.

Die zugrundeliegende Aufgabe besteht also in der Verbesserung der Reinigungsleistung herkömmlicher Haarpflege- oder Haarreinigungsproduke, insbesondere im Hinblick auf das Entfernen von Ablagerungen von blondem, insbesondere blondiertem, Haar. Diese wird von dem hier beschriebenen Haarpflegebooster gemäß der Erfindung hervorragend gelöst. Es zeigte sich außerdem, dass unerwarteter weise ein positiver Einfluss auf die innere Struktur, nämlich eine Stärkung dieser inneren Struktur, des behandelten Haars nachgewiesen werden konnte. Die der Erfindung zugrundeliegende Aufgabe ist auch die Verbesserung der inneren Struktur des Haars.

Diese Aufgaben werden gelöst durch einen ersten Aspekt der Erfindung, einen Haarpflegebooster in Pulverform, der
- 50 Gew.-% bis 79 Gew.-% einer organischen Carbonsäure;
- eine proteinogene Aminosäure aus der Gruppe Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin;
- ein Purin oder Purin-Abbauprodukte, aus der Gruppe Adenin, Guanin, Harnsäure, Allantoin und Hypoxanthin;
- Aminosäure-Abbauprodukte aus der Gruppe Taurin; und
- ein Alkalisierungsmittel umfasst. Eine portioniere Menge dieses Boosters wird auch als "Shot" oder "Booster Shot" bezeichnet und diese Begriffe können synonym verwendet werden. Der Booster ist dafür vorgesehen, einem herkömmlichen Haarpflege-produkt, insbesondere einer Haarkur kurz vor deren Anwendung zugesetzt werden. In anderen Worten handelt es sich um ein Additiv oder eine Additiv-Mischung, auch Additiv-Blend genannt.

Die organische Carbonsäure ist nicht sonderlich beschränkt, ist aber bevorzugt eine Carbonsäure mit höchsten sieben, weiterhin bevorzugt höchstens sechs, insbesondere bevorzugt höchstens vier Kohlenstoffatomen. Die organische Carbonsäure kann auch eine vinyloge Carbonsäure wie Ascorbinsäure sein. Insbesondere bevorzugt ist die organische Carbonsäure eine von Ascorbinsäure, Ameisensäure, Äpfelsäure, Citronensäure, Essigsäure, Benzoesäure, Oxalsäure, Maleinsäure oder ein Gemisch derselben. Diese Carbonsäuren sind von eher geringer Molekülgrüße, sodass sie gute Wasserlöslichkeiten aufweisen und ihre Carbonsäureeigenschaften im Vergleich zu bei größeren Molekülen möglicherweise in den Vordergrund tretenden anderen Eigenschaften prononciert sind. Damit lässt sich die zuvor beschriebene vorteilhafte Wirkung der Erfindung in besonders ausgeprägten Ausmaß herbeiführen.

Der Haarpflegebooster in Pulverform umfasst, bezogen auf sein Gesamtgewicht, 50 Gew.-% bis 79 Gew.-%, bevorzugt 60 Gew.-% bis 74 Gew.-%, insbesondere bevorzugt 55 Gew.-% bis 70 Gew.-% der organische Carbonsäure. Es hat sich herausgestellt, dass ein Haarpflegebooster in Pulverform, in dem die Konzentration der organischen Carbonsäure in diesen Bereichen liegt, die bereits erwähnten vorteilhaften Wirkungen in zunehmendem Ausmaß aufweist und mithin die der Erfindung zugrunde liegenden Aufgaben besonders gut löst. In dem Haarpflegebooster in Pulverform kann als die organische Carbonsäure auch ein Gemisch verschiedener organischer Carbonsäuren vorliegen.

Der Haarpflegebooster in Pulverform umfasst bevorzugt, bezogen auf sein Gesamtgewicht, 5 Gew.-% bis 15 Gew.-%, weiterhin bevorzugt 8 Gew.-% bis 13 Gew.-%, insbesondere bevorzugt 9 Gew.-% bis 12 Gew.-% der proteinogenen Aminosäure. Es hat sich herausgestellt, dass ein Haarpflegebooster in Pulverform, in dem die Konzentration der proteinogenen Aminosäure in diesen Bereichen liegt, die bereits erwähnten vorteilhaften Wirkungen in zunehmendem Ausmaß aufweist und mithin die der Erfindung zugrunde liegenden Aufgaben besonders gut löst. In dem Haarpflegebooster in Pulverform kann als die proteinogene Aminosäure auch ein Gemisch verschiedener proteinogener Aminosäuren vorliegen.

Proteinogene Aminosäuren im Sinne der Erfindung sind Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin.

Der Haarpflegebooster in Pulverform umfasst bevorzugt, bezogen auf sein Gesamtgewicht, 2 Gew.-% bis 10 Gew.-%, weiterhin bevorzugt 3 Gew.-% bis 8 Gew.-%, insbesondere bevorzugt 4 Gew.-% bis 6 Gew.-% Purin oder Purin-Abbauprodukte. Es hat sich herausgestellt, dass ein Haarpflegebooster in Pulverform, in dem die Konzentration des Purins oder der Purin-Abbauprodukte in diesen Bereichen liegt, die bereits erwähnten vorteilhaften Wirkungen in zunehmendem Ausmaß aufweist und mithin die der Erfindung zugrunde liegenden Aufgaben besonders gut löst. Als Purin oder Purin-Abbauprodukte können hier auch verschiedene Purine und/oder Purin-Abbauprodukte als Gemisch vorliegen. Purine oder Purin Abbauprodukte im Sinne der Erfindung sind Adenin, Guanin, Harnsäure, Allantoin und Hypoxanthin, mit denen sich die zuvor beschriebene erfindungsgemäße, technisch vorteilhafte Wirkung in besonders ausgeprägtem Ausmaß herbeiführen lässt.

Der Haarpflegebooster in Pulverform umfasst bevorzugt, bezogen auf sein Gesamtgewicht, Gew.-% bis 15 Gew.-%, weiterhin bevorzugt 8 Gew.-% bis 13 Gew.-%, insbesondere bevorzugt 9 Gew.-% bis 12 Gew.-% Aminosäure-Abbauprodukte. Es hat sich herausgestellt, dass ein Haarpflegebooster in Pulverform, in dem die Konzentration der Aminosäure-Abbauprodukte in diesen Bereichen liegt, die bereits erwähnten vorteilhaften Wirkungen in zunehmendem Ausmaß aufweist und mithin die der Erfindung zugrunde liegenden Aufgaben besonders gut löst. Als Aminosäureabbauprodukt im Sinne der Erfindung wird Taurin verwendet, Aminosulfonsäure ist.

Der Haarpflegebooster in Pulverform umfasst bevorzugt, bezogen auf sein Gesamtgewicht, 1 Gew.-% bis 8 Gew.-%, weiterhin bevorzugt 2 Gew.-% bis 6 Gew.-%, insbesondere bevorzugt 3 Gew.-% bis 5 Gew.-% Alkalisierungsmittel. Es hat sich herausgestellt, dass ein Haarpflegebooster in Pulverform, in dem die Konzentration des Alkalisierungsmittel in diesen Bereichen liegt, die bereits erwähnten vorteilhaften Wirkungen in zunehmendem Ausmaß aufweist und mithin die der Erfindung zugrunde liegenden Aufgaben besonders gut löst.

Das Alkalisierungsmittel ist nicht sonderlich beschränkt und kann jedes für kosmetische zwecke akzeptable Alkalisierungsmittel sein. Bevorzugt ist es aber ein anorganisches Hydroxid, weiterhin bevorzugt ein Alkali oder Erdalkalihydroxid, von diesen bevorzugt ein Erdalkalihydroxid, insbesondere bevorzugt Calciumhydroxid. Es hat sich gezeigt, dass bei solchen Auswahlen die zuvor beschriebenen, technisch vorteilhaften Wirkungen der Erfindung in zunehmendem Ausmaß hervortreten.

Außerdem Gegenstand des ersten Aspekts der Erfindung ist ein Kosmetisches Produkt, das den Haarpflegebooster in Pulverform und ein separat von diesem bereitgestelltes Haarpflegeprodukt, insbesondere eine Haarkur, umfasst bei dessen Anwendung die zuvor beschriebenen Vorteile erzielt werden. Die für den ersten Aspekt beschriebenen Merkmale können sich, sofern kein besonderer Hinderungsgrund vorliegt auch im zweiten Aspekt der Erfindung wiederfinden.

Der zweite Aspekt ist ein Verfahren, das die folgenden Schritte umfasst: Vermischen Haarpflegeboosters in Pulverform gemäß des ersten Aspekts mit einem Haarpflegeprodukt, insbesondere einer Haarkur, und Erhalten eines anwendungsfertigen Gemischs. Bevorzugt umfassend das Verfahren den Schritt: Anwenden des anwendungsfertigen Gemischs an keratinischen Fasern, insbesondere menschlichen Haaren. Weiterhin bevorzugt umfasst das Verfahren die Schritte: Belassen des anwendungsfertigen Gemischs für 10 Sekunden bis 20 Minuten an den keratinischen Fasern, insbesondere menschlichen Haaren, und Ausspülen des anwendungsfertigen Gemischs. Es ist bevorzugt, dass der Schritt des Vermischens eher kurz ist und vier Minuten nicht überschreitet, die Zeitspanne kann insbesondere bevorzugt in einem Bereich von ein bis drei Minuten, zum Beispiel bei zwei Minuten liegen. Weiterhin ist es bevorzugt, dass die Zeitspanne zwischen dem Vermischen und dem Anwenden an keratinischen Fasern nicht zu lang ist. Die Zeitspanne zwischen dem Vermischen und dem Anwenden an keratinischen Fasern kann beispielsweise in einem Bereich von 10 bis 15 Minuten liegen.

Diese vorteilhaften Weiterbildungen ermöglichen es, dass die bereits erwähnten vorteilhaften Wirkungen in zunehmendem Ausmaß erzielt werden und mithin die der Erfindung zugrunde liegenden Aufgaben besonders gut gelöst wird.

Ein konkretes Ausführungsbeispiel des ersten Aspekts der Erfindung ist die Folgende Booster-Shot-Zusammensetzung:

| Bestandteil | Anteil in Gewichtsprozent |
|---|---|
| Vitamin C, DAB | 55,60 |
| Citronensäure, wasserfrei, fein granuliert | 4,80 |
| Allantoin, USP | 5,30 |
| Lysin HCl | 12,00 |
| Taurin | 12,00 |
| Äpfelsäure, DL | 5,30 |
| Calciumhydroxid | 5,00 |
| Gesamt | 100,00 |

## Patentansprüche

1. Haarpflegebooster in Pulverform, umfassend:
- 50 Gew.-% bis 79 Gew.-% einer organischen Carbonsäure,
- eine proteinogene Aminosäure aus der Gruppe Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin,
- ein Purin oder Purin-Abbauprodukte, aus der Gruppe Adenin, Guanin, Harnsäure, Allantoin und Hypoxanthin
- Aminosäure-Abbauprodukte aus der Gruppe Taurin, und
- ein Alkalisierungsmittel.

2. Haarpflegebooster in Pulverform nach Anspruch 1, wobei die organische Carbonsäure 60 Gew.-% bis 74 Gew.-%, insbesondere bevorzugt 55 Gew.-% bis 70 Gew.-% des Haarpflegeboosters in Pulverform insgesamt ausmacht.

3. Haarpflegebooster in Pulverform nach Anspruch 1 oder 2, wobei die proteinogene Aminosäure 5 Gew.-% bis 15 Gew.-%, bevorzugt 8 Gew.-% bis 13 Gew.-%, insbesondere bevorzugt 9 Gew.-% bis 12 Gew.-% des Haarpflegeboosters in Pulverform insgesamt ausmacht.

4. Haarpflegebooster in Pulverform nach einem der Ansprüche 1 bis 3, wobei das Purin oder Purin-Abbauprodukte 2 Gew.-% bis 10 Gew.-%, bevorzugt 3 Gew.-% bis 8 Gew.-%, insbesondere bevorzugt 4 Gew.-% bis 6 Gew.-% des Haarpflegeboosters in Pulverform insgesamt ausmachen.

5. Haarpflegebooster in Pulverform nach einem der Ansprüche 1 bis 4, wobei Aminosäure-Abbauprodukte 5 Gew.-% bis 15 Gew.-%, bevorzugt 8 Gew.-% bis 13 Gew.-%, insbesondere bevorzugt 9 Gew.-% bis 12 Gew.-% des Haarpflegeboosters in Pulverform insgesamt ausmachen.

6. Haarpflegebooster in Pulverform nach einem der Ansprüche 1 bis 5, wobei ein Alkalisierungsmittel 1 Gew.-% bis 8 Gew.-%, bevorzugt 2 Gew.-% bis 6 Gew.-%, insbesondere bevorzugt 3 Gew.-% bis 5 Gew.-% des Haarpflegeboosters in Pulverform insgesamt ausmacht.

7. Kosmetisches Produkt, umfassend:
den Haarpflegebooster in Pulverform nach einem der Ansprüche 1 bis 6, und
ein separat von diesem bereitgestelltes Haarpflegeprodukt, insbesondere eine Haarkur.

8. Verfahren, umfassend die Schritte:
- Vermischen eines Haarpflegeboosters in Pulverform nach einem der Ansprüche 1 bis 6 mit einem Haarpflegeprodukt, insbesondere einer Haarkur, und
- Erhalten eines anwendungsfertigen Gemischs.

9. Verfahren nach Anspruch 8, ferner umfassend den Schritt:
Anwenden des anwendungsfertigen Gemischs an keratinischen Fasern, insbesondere menschlichen Haaren.

10. Verfahren nach Anspruch 9, ferner umfassend die Schritte:
Belassen des anwendungsfertigen Gemischs für 10 Sekunden bis 20 Minuten an den keratinischen Fasern, insbesondere menschlichen Haaren, und
Ausspülen des anwendungsfertigen Gemischs.

## Claims

1. Hair care booster in powder form, comprehensive:
- 50 % to 79 % by weight of an organic carboxylic acid,
- a proteinogenic amino acid from the group alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine,
- a purine or purine degradation products, from the group adenine, guanine, uric acid, allantoin and hypoxanthine
- amino acid degradation products from the taurine group, and
- an alkalizing agent.

2. Hair care booster in powder form according to claim 1, wherein the organic carboxylic acid makes up 60 wt.% to 74 wt.%, in particular preferably 55 wt.% to 70 wt.% of the hair care booster in powder form as a whole.

3. Hair care booster in powder form according to claim 1 or 2, wherein the proteinogenic amino acid makes up 5 wt.% to 15 wt.%, preferably 8 wt.% to 13 wt.%, more preferably 9 wt.% to 12 wt.% of the hair care booster in powder form in total.

4. Hair care booster in powder form according to any one of claims 1 to 3, wherein the purine or purine degradation products account for 2 wt.% to 10 wt.%, preferably 3 wt.% to 8 wt.%, more preferably 4 wt.% to 6 wt.% of the hair care booster in powder form in total.

5. Hair care booster in powder form according to any one of claims 1 to 4, wherein amino acid degradation products account for 5% by weight to 15% by weight, preferably 8% by weight to 13% by weight, more preferably 9% by weight to 12% by weight of the hair care booster in powder form in total.

6. Hair care booster in powder form according to any one of claims 1 to 5, wherein an alkalizing agent accounts for 1 wt.% to 8 wt.%, preferably 2 wt.% to 6 wt.%, more preferably 3 wt.% to 5 wt.% of the hair care booster in powder form in total.

7. Cosmetic product, comprehensive:
the hair care booster in powder form according to any one of claims 1 to 6, and
a hair care product provided separately from this, in particular a hair conditioner.

8. A method comprising the steps of:
- Mixing a hair care booster in powder form according to one of claims 1 to 6 with a hair care product, in particular a hair conditioner, and
- Obtaining a ready-to-use mixture.

9. the method according to claim 8, further comprising the step of:
Apply the ready-to-use mixture to keratin fibers, especially human hair.

10. the method according to claim 9, further comprising the steps of:
Leaving the ready-to-use mixture on the keratinous fibers, in particular human hair, for 10 seconds to 20 minutes, and
Rinse out the ready-to-use mixture.

## Revendications

1. Booster de soins capillaires sous forme de poudre, comprenant:
- 50 % à 79 % en poids d'un acide carboxylique organique,
- un acide aminé protéinogène choisi parmi l'alanine, l'arginine, l'asparagine, l'acide aspartique, la cystéine, la glutamine, l'acide glutamique, la glycine, l'histidine, l'isoleucine, la leucine, la lysine, la méthionine, la phénylalanine, la proline, la sérine, la thréonine, le tryptophane, la tyrosine et la valine,
- une purine ou des produits de dégradation de la purine, choisis parmi l'adénine, la guanine, l'acide urique, l'allantoïne et l'hypoxanthine
- les produits de dégradation des acides aminés du groupe de la taurine, et
- un agent alcalinisant.

2. Booster de soins capillaires sous forme de poudre selon la revendication 1, dans lequel l'acide carboxylique organique représente 60% à 74% en poids, de préférence 55% à 70% en poids du total du booster de soins capillaires sous forme de poudre.

3. Booster de soins capillaires sous forme de poudre selon la revendication 1 ou 2, dans lequel l'acide aminé protéinogène représente 5 % à 15 % en poids, de préférence 8 % à 13 % en poids, en particulier de préférence 9 % à 12 % en poids du total du booster de soins capillaires sous forme de poudre.

4. Booster de soin capillaire sous forme de poudre selon l'une quelconque des revendications 1 à 3, dans lequel la purine ou les produits de dégradation de la purine représentent 2 % à 10 % en poids, de préférence 3 % à 8 % en poids, en particulier de préférence 4 % à 6 % en poids du total du booster de soin capillaire sous forme de poudre.

5. Booster de soins capillaires sous forme de poudre selon l'une quelconque des revendications 1 à 4, dans lequel les produits de dégradation des acides aminés représentent 5 % à 15 % en poids, de préférence 8 % à 13 % en poids, en particulier de préférence 9 % à 12 % en poids du total du booster de soins capillaires sous forme de poudre.

6. Booster de soins capillaires sous forme de poudre selon l'une quelconque des revendications 1 à 5, dans lequel un agent alcalinisant représente de 1 % à 8 % en poids, de préférence de 2 % à 6 % en poids, en particulier de préférence de 3 % à 5 % en poids du total du booster de soins capillaires sous forme de poudre.

7. Produit cosmétique, complet:
le booster de soin capillaire sous forme de poudre selon l'une quelconque des revendications 1 à 6, et
un produit de soin capillaire fourni séparément de celui-ci, en particulier un traitement capillaire.

8. Procédé comprenant les étapes consistant à:
- Mélange d'un booster de soin capillaire sous forme de poudre selon l'une quelconque des revendications 1 à 6 avec un produit de soin capillaire, en particulier un traitement capillaire, et
Obtenir un mélange prêt à l'emploi.

9. Procédé selon la revendication 8, comprenant en outre l'étape consistant à:
Appliquer le mélange prêt à l'emploi sur les fibres kératiniques, en particulier les cheveux humains.

10. Procédé selon la revendication 9, comprenant en outre les étapes consistant à:
laisser le mélange prêt à l'emploi sur les fibres kératiniques, en particulier les cheveux humains, pendant 10 secondes à 20 minutes, et
Rincer le mélange prêt à l'emploi.
